# EUROPÄISCHE PATENTSCHRIFT

(11) **EP 1 377 591 B1**
(45) Veröffentlichungstag und Bekanntmachung des Hinweises auf die Patenterteilung: **28.06.2006**
(21) Anmeldenummer: 02708371.6
(22) Anmeldetag: 28.03.2002
(51) Int. Cl.: C07F 5/00

(54) **TUMORHEMMENDE GALLIUMVERBINDUNGEN**
TUMOUR INHIBITING GALLIUM COMPOUNDS
COMPOSES DE GALLIUM ANTITUMORAUX

(30) Priorität: 03.04.2001 DE 10116527
(43) Veröffentlichungstag der Anmeldung: 07.01.2004
(73) Patentinhaber: Faustus Forschungs Cie. Translational Cancer Research GmbH, 04109 Leipzig (DE)
(72) Erfinder: KEPPLER, Bernhard, 68766 Hockenheim (DE)
(74) Vertreter: Grünecker, Kinkeldey, Stockmair & Schwanhäusser Anwaltssozietät
(86) Internationale Anmeldenummer: PCT/EP2002/003560
(87) Internationale Veröffentlichungsnummer: WO 2002/081484

(56) Entgegenhaltungen:
- US-A- 4 596 710
- US-A- 4 657 903
- US-A- 5 484 778
- KLAYMAN, D. L. ET AL: "2-Acetylpyridine thiosemicarbazones. 6. 2-Acetylpyridine and 2-butyrylpyridine thiosemicarbazones as antileukemic agents" ARZNEIM.-FORSCH. (1983), 33(7), 909-12 , XP001071299
- HALL, IRIS H. ET AL: "The cytotoxicity of heterocyclic thiosemicarbazones and their metal complexes on human and murine tissue culture cells" ANTI-CANCER DRUGS (1993), 4(2), 231-40 , XP002079621
- KOVALA-DEMERTZI, DIMITRA ET AL: "Palladium(II) complexes of 2-acetylpyridine N(4)-propyl-, N(4)-dipropyl- and 3-hexamethyleneiminylthiosemicarbazones with potentially interesting biological activity. Synthesis, spectral properties, antifungal and in vitro antitumor activity" POLYHEDRON (1997), 16(20), 3625-3633 , XP002201201

## Beschreibung

Die vorliegende Erfindung betrifft Galliumverbindungen sowie deren Verwendung als Arzneimittel zur Prophylaxe und/oder Behandlung von Krebserkrankungen.

In der US-A-6 087 354 werden pharmazeutische Zusammensetzungen beschrieben, die Gallium-Komplexe des 3-Hydroxy-4-pyrons umfassen. Die Zusammensetzungen sind für eine Vielzahl medizinischer Anwendungen, wie die Behandlung von Krebserkrankungen, geeignet.

Die US-A-5 484 778 beschreibt Gallium-Phthalocyanine und deren Verwendung zur Behandlung von Krebserkrankungen.

In der US-A-4 596 710 wird die Verwendung von Galliumchlorid zur Behandlung von malignen Tumoren beschrieben.

Die EP-A-0525 938 beschreibt Gallium(III)-Komplexe und deren Verwendung zur Behandlung von Hypercalcämie und von Krebserkrankungen.

Weiterhin ist in der WO-A-9 302 087 die tumorhemmende Wirkung der Komplex-verbindung Tris(8-chinolinolato)-gallium(III) beschrieben.

2-Acetylpyridin- und 2-Butyrylpyridin-thiosemicarbazone mit antileukämischer Aktivität werden in *Arzneim.-Forsch.* (1983), 33(7), S. 909-12 offenbart.

Die US 4,657,903 offenbart 2-Acetyl- und 2-Propionyl-Pyridinthiosemicarbazone, deren Selen-Analoge sowie die antileukämische Aktivität und Antimalaria-Aktivität der Verbindungen.

Die Zytotoxizität von heterocyclischen Thiosemicarbazonen und deren Metallkomplexen auf menschliche und murine Gewebezellkulturen wird in *Anti-Cancer Drugs* (1993), 4(2), S. 231-40 beschrieben.

In *Polyhedron,* Vol.16, Nr. 20, S. 3625-3633, 1997 werden Palladium(II)-Komplexe von 2-Acetylpyridin N(4)-propyl-, N(4)-dipropyl- und 3-Hexamethyleniminylthiosemicarbazonen mit *in vitro* Antitumoraktivität beschrieben.

Aufgabe der vorliegenden Erfindung ist es, Verbindungen zur Verfügung zu stellen, die eine hohe Wirksamkeit zur Behandlung von Krebserkrankungen aufweisen.

Diese Aufgabe wird durch eine Verbindung der allgemeinen Formel (I) 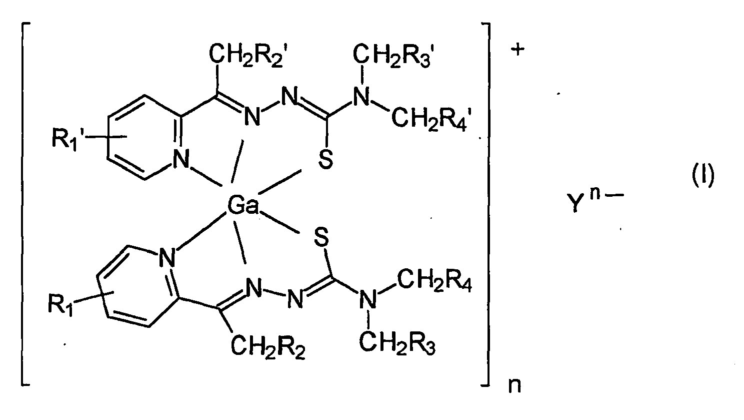 worin
- R₁, R₂, R₃, R₄, R₁', R₂', R₃' und R₄': unabhängig voneinander Wasserstoff, C₁-C₁₅-Alkyl, C₂-C₁₅-Alkenyl, C₂-C₁₅-Alkinyl, C₃-C₁₆-Cycloalkyl, C₃-C₁₆-Cycloalkenyl, Aryl oder ein Heterocyclus, die jeweils substituiert oder unsubstituiert sein können, ist,
- Y: ein physiologisch verträgliches Anion, vorzugsweise ein Metallhalogen, Halogenoborat, Halogen, Pseudohalogen, HCO₃, oder R'COO, worin R' C₁-C₁₅-Alkyl, C₂-C₁₅-Alkenyl, C₂-C₁₅-Alkinyl, C₃-C₁₆₋Cycloalkyl, C₃-C₁₆-Cycloalkenyl oder Aryl, die jeweils substituiert oder unsubstituiert sein können, ist, und
- n: eine natürliche Zahl, vorzugsweise 1 bis 3, bevorzugter 1 oder 2, insbesondere 1, ist,
gelöst.

In einer bevorzugten Ausführungsform sind
- R₁, R₂, R₃, R₄, R₁', R₂', R₃' und R₄': unabhängig voneinander Wasserstoff, C₁-C₁₀-Alkyl, C₂-C₁₀-Alkenyl, C₂-C₁₀-Alkinyl,- C₃-C₁₀-Cycloalky, C₃-C₁₀-Cycloalkenyl, Aryl oder ein Heterocyclus, die jeweils substituiert oder unsubstituiert sein können, und
- Y: ein Metallhalogen, Halogenoborat, Halogen, Pseudohalogen, HCO₃, oder R'COO, worin R' C₁-C₁₀-Alkyl, C₂-C₁₀-Alkenyl, C₂-C₁₀-Alkinyl, C₃-C₁₀-Cycloalkyl, C₃-C₁₀-Cycloalkenyl oder Aryl, die jeweils substituiert oder unsubstituiert sein können.

In einer besonders bevorzugten Ausführungsform sind
- R₁, R₂, R₃, R₄, R₁', R₂', R₃' und R₄': unabhängig voneinander Wasserstoff, C₁ - C₆ -Alkyl, C₂ - C₆ -Alkenyl, C₂-C₆-Alkinyl, C₃ - C₆ -Cycloalkyl, C₃-C₆-Cycloalkenyl, C₆ - C₁₄-Aryl oder ein Heterocyclus, die jeweils substituiert oder unsubstituiert sein können, und
- Y: ein Metallhalogen, Halogen, Pseudohalogen, HCO₃ oder R'COO, worin R' C₁ - C₆ -Alkyl, C₂ - C₆ -Alkenyl, C₂-C₆-Alkinyl, C₃-C₆₋Cycloalkyl, C₃-C₆-Cycloalkenyl oder Aryl, die jeweils substituiert oder unsubstituiert sein können.

Das Halogen ist vorzugsweise Fluor, Chlor, Brom oder lod, bevorzugt Fluor, Chlor oder Brom, und insbesondere Chlor.

Das Metall des Metallhalogens kann entweder aus den Haupt- oder Nebengruppen stammen, bevorzugt aus der 2., 3., 4. oder 5. Hauptgruppe, besonders bevorzugt aus der 3. Hauptgruppe, und ganz besonders bevorzugt ist das Metall Gallium.

Das Halogen des Metallhalogens ist vorzugsweise wie vorstehend für Halogen definiert.

In einer bevorzugten Ausführungsform ist Y in der allgemeinen Formel (1) ein Galliumhalogen, ganz besonders bevorzugt [GaCl₄].

Ferner wird die Aufgabe der vorliegenden Erfindung durch ein Arzneimittel gelöst, das die erfindungsgemäße Verbindung enthält. Die erfindungsgemäße Verbindung kann zur Prophylaxe und/oder Behandlung von Krebserkrankungen eingesetzt werden.

Im folgenden wird das Arzneimittel, enthaltend eine erfindungsgemäße Verbindung, genauer beschrieben.

Das erfindungsgemäße Arzneimittel wird vor ahem intravenöse aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Das Arzneimittel wird nach an sich bekannten Verfahren hergestellt, wobei die erfindungsgemäße Verbindung als solche oder gegebenenfalls in Kombination mit geeigneten pharmazeutischen Trägerstoffen eingesetzt wird. Enthält das erfindungsgemäße Arzneimittel neben dem Wirkstoff pharmazeutische Trägerstoffe, beträgt der Wirkstoffgehalt dieser Mischung 0,1 bis 99,5, vorzugsweise 0,5 bis 95 Gew. % der Gesamtmischung.

Das erfindungsgemäße Arzneimittel kann in jeder geeigneten Formulierung angewandt werden unter der Voraussetzung, dass die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter , Einheitsdosis" im Sinne der vorliegenden Erfindung wird eine physikalisch bestimmte Einheit verstanden, die eine individuelle Menge des aktiven Bestandteils in Kombination mit einem pharmazeutischen Trägerstoff enthält und deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheltsdosis vorteilhafterweise teilbar, z.B. in Form einer Tablette mit Bruchkerbe.

Die erfindungsgemäßen Arzneimittel können, wenn sie in Einheitsdosen vorliegen und für Applikationen z.B. am Menschen bestimmt sind, etwa 0,1 bis 500 mg, bevorzugt 10 bis 200 mg und insbesondere 50 bis 150 mg Wirkstoff enthalten.

Im allgemeinen werden in der Humanmedizin der oder die Wirkstoffe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht. Bei einer oralen Behandlung können ähnliche Dosierungen zur Anwendung kommen.

Die therapeutische Verabreichung des erfindungsgemäßen Arzneimittels kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Es kann sich auch als zweckmäßig erweisen, die Arzneimittel nur einmalig oder im Abstand von mehreren Tagen zu verabreichen.

Die Festlegung der erforderlichen optimalen Dosierung und Applikationsart der Wirkstoffe kann durch jeden Fachmann aufgrund seines Fachwissens erfolgen.

Die erfindungsgemäßen Arzneimittel bestehen in der Regel aus den erfindungsgemäßen Verbindungen und nichttoxischen, pharmazeutisch verträglichen Arzneimittelträgern, die als Zumischung oder Verdünnungsmittel, beispielsweise in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten Dragees, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Laktose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption der Arzneimittelzubereitung im Gastrointestinaltrakt bewirkt, so dass z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuss-, oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die erfindungsgemäße Verbindung in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuss-, oder Paraffinöl neben Emulgiermitteln, wie z. B. Akaziengummi, Traganthgummi, Phosphatiden, Sorbitanmonooleat, Polyoxyethylensorbitanmonooleat, und Süßungs- und Geschmacksmittel enthalten.

Wässrige Lösungen können Konservierungsmittel, z.B. Methyl- oder Propylhydroxybenzoate; Verdickungsmittel; Geschmacksmittel; Süßungsmittel, z.B. Saccharose, Laktose, Natriumcyclamat, Dextrose, Invertzuckersirup, sowie Geschmacksmittel und Farbstoffe enthalten.

Zur parenteralen Anwendung der Arzneistoffe dienen steril injizierbare, wässrige Lösungen, isotonische Salzlösungen oder sonstige Lösungen.

Die folgenden Beispiele erläutern die Erfindung.

### Beispiel 1

### Synthese von Bis(2-acetylpyridin-4,4-dimethylthiosemicarbazonato-N1,N2,S)gallium(III)-tetrachlorogallat(III)

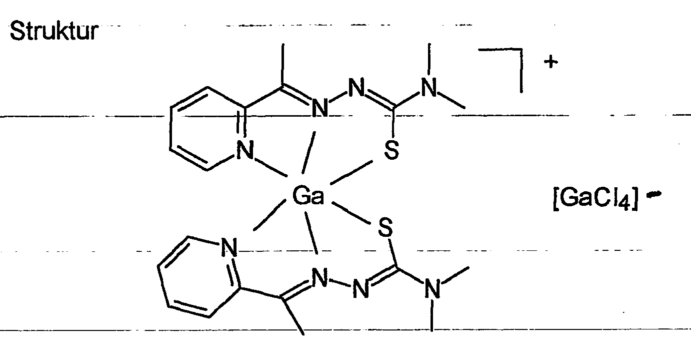

Die Herstellung erfolgt durch Umsetzung von 2-Acetylpyridin-4,4-dimethylthiosemicarbazon (DATSC) mit Gallium(III)chlorid (GaCl₃) im Verhältnis DATSC : GaCl₃ = 1 : 1. Hierbei wird der Ligand bei Raumtemperatur in absolutem Ethanol gelöst und anschließend die (ethanolische) GaCl₃-Lösung innerhalb von 5-10 Minuten zugetropft. Die unter Rühren entstehende Suspension des ausfallenden Produkts wird nach einer Stunde abgesaugt, mit wenig absolutem Ethanol sowie viel absolutem Ether gewaschen, aus absolutem Ethanol umkristallisiert und im Hochvakuum getrocknet.

### Analyse:

| | | | | | | |
|---|---|---|---|---|---|---|
| Berechnet: | C: 33,19 | H: 3,62 | Cl: 19,60 | N:15,48 | 5: 8,86 | Ga: 19,26 |
| Gefunden: | C. 33,28 | H: 3,67 | Cl: 19,45 | N: 15,22 | 5: 8,78 | Ga: 19,60 |

### Beispiel 2

### Tumorhemmende Aktivität von Bis(2-acetylpyridin-4,4-dimethylthiosemicarbazonato-N1,N2,S)gallium(III)-tetrachlorogallat(III)

Testungen an zwei murinen-Tamorzellinien unter-96-stündiger Exposition zeigten eine außerordentlich hohe tumorhemmende Aktivität (bereits im nanomolaren Bereich):
MAC15A (murines Adenokarzinom des Kolons):
IC₅₀: 0,11 nmol/l 0,08 ng/ml
F9 (murines Teratokarzinom des Hodens):
IC₅₀; 17,4 nmol/l 12,6 ng/ml

Weiterhin zeigte sich eine sehr markante Selektivität für Mamma-, Prostata- und großzelliges Bronchialkarzinom (IC₅₀ < 0.003 mg/ml). Vergleichbare Effekte waren auch an je einem Magenkarzinom- und Melanom-Xenograft sowie an einer Ovarial-(OVCAR3) und einer kleinzelligen Bronchialkarzinomzellinie (DMS 114) festzustellen. Eine überdurchschnittliche Aktivität wurde zudem an einem von zwei Nierenzellkarzinom-Xenografts beobachtet.

## Patentansprüche

1. Verbindung der allgemeinen Formel (I) 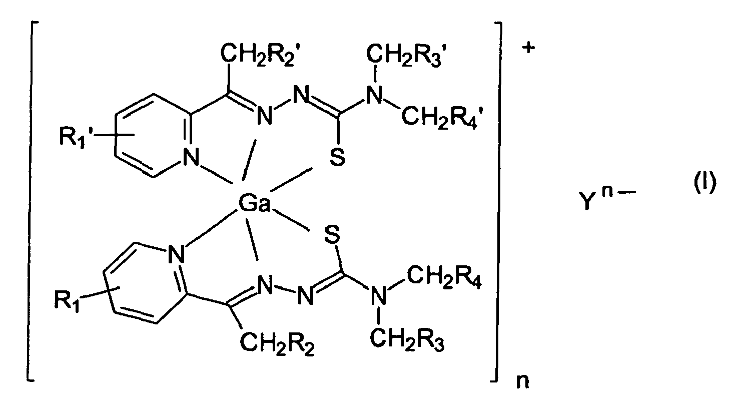 worin
R₁, R₂, R₃, R₄, R₁', R₂', R₃' und R₄' unabhänig voneinander Wasserstoff, C₁-C₁₅-Alkyl, C₂-C₁₅-Alkenyl, C₂-C₁₅-Alkinyl, C₃-C₁₆-Cycloalkyl, C₃-C₁₆-Cycloalkenyl, Aryl oder ein Heterocyclus, die jeweils substituiert oder unsubstituiert sein können, ist,
Y ein physiologisch verträgliches Anion, ist, und
n eine natürliche Zahl ist.

2. Verbindung nach Anspruch 1, worin Y [GaCl₄] ist.

3. Arzneimittel, enthaltend eine Verbindung der allgemeinen Formel (I) nach mindestens einem der Ansprüche 1 oder 2.

4. Verwendung einer Verbindung der allgemeinen Formel (I) nach mindestens einem der Ansprüche 1 oder 2 zur Herstellung eines Medikaments zur Prophylaxe und/oder Behandlung von Krebserkrankungen.

## Claims

1. Compound of the general formula (I) 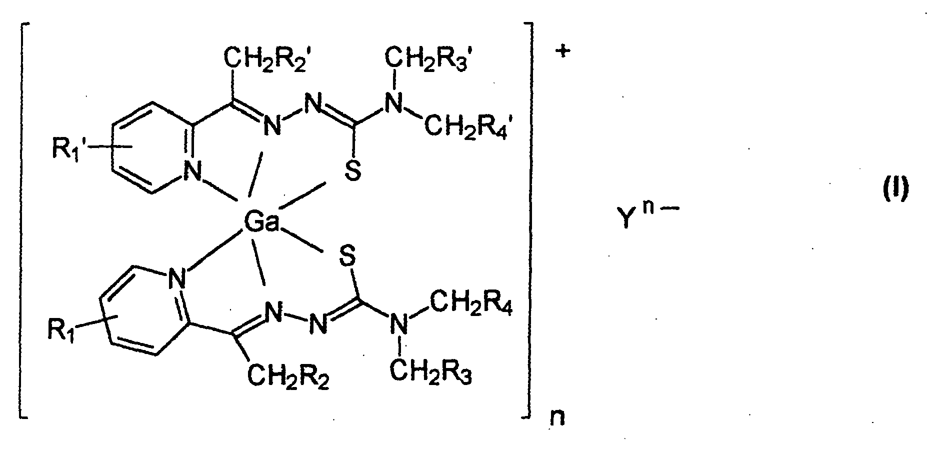 where
R₁, R₂, R₃, R₄, R₁', R₂', R₃', and R₄' are independently of one another hydrogen, C₁-C₁₅-alkyl, C₂-C₁₅-alkenyl, C₂-C₁₅₋alkinyl, C₃-C₁₆-cycloalkyl, C₃-C₁₆-cycloalkenyl, aryl or a heterocyclus which in each case can be substituted or unsubstituted,
Y is a physiologically compatible anion, and
n is a natural number.

2. Compound according to claim 1, where Y is [GaCl₄].

3. Medicament containing a compound of the general formula (I) according to at least one of claims 1 or 2.

4. Use of a compound of the general formula (I) according to at least one of the claims 1 or 2 for the preparation of a medicament for the prophylaxis and/or treatment of cancer illnesses.

## Revendications

1. Composé de formule générale (I) : 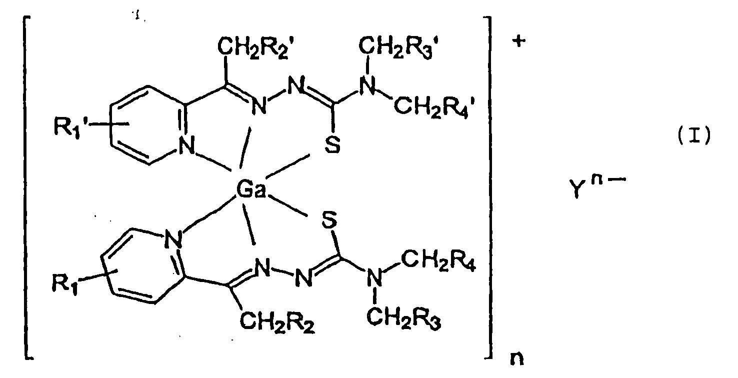 dans laquelle
R₁, R₂, R₃, R₄, R'₁, R'₂, R'₃, R'₄
représentent, indépendamment les uns des autres, hydrogène, alkyle en C₁-C₁₅, alcényle en C₂-C₁₅, alcinyle en C₂-C₁₅, cycloalkyle en C₃-C₁₆, cycloalcényle en C₃-C₁₆, aryle ou hétérocycle, qui peuvent chacun être substitués ou non substitués,
Y représente un anion physiologiquement acceptable, et
n est un nombre naturel.

2. Composé selon la revendication 1, dans lequel Y est [Ga Cl₄] .

3. Médicament contenant un composé de formule générale (I) selon au moins l'une des revendications 1 et 2.

4. Utilisation d'un composé de formule générale (I) selon au moins l'une des revendications 1 et 2, pour la préparation d'un médicament pour la prophylaxie et/ou le traitement d'une maladie cancéreuse.
